# EUROPÄISCHE PATENTSCHRIFT

(11) **EP 0 991 377 B1**
(45) Veröffentlichungstag und Bekanntmachung des Hinweises auf die Patenterteilung: **19.09.2001**
(21) Anmeldenummer: 99924968.3
(22) Anmeldetag: 11.05.1999
(51) Int. Cl.: A61F 2/08

(54) **VORRICHTUNG ZUR BEFESTIGUNG EINES ALLOPLASTISCHEN BANDES AM KNOCHEN**
DEVICE FOR FIXING AN ALLOPLASTIC BAND ON A BONE
DISPOSITIF POUR FIXER UNE BANDE ALLOPLASTIQUE SUR UN OS

(30) Priorität: 10.06.1998 DE 19825997
(43) Veröffentlichungstag der Anmeldung: 12.04.2000
(73) Patentinhaber: Huber, Jacob, 82327 Tutzing (DE)
(72) Erfinder: Huber, Jacob, 82327 Tutzing (DE)
(74) Vertreter: Lewald, Dietrich, Dipl.-Ing.
(86) Internationale Anmeldenummer: EP9903315
(87) Internationale Veröffentlichungsnummer: WO9963911

(56) Entgegenhaltungen:
- EP-A- 0 425 140
- WO-A-97/29706
- WO-A-97/36557
- DE-A- 19 629 011
- US-A- 5 702 445

## Beschreibung

Die erfindung betrifft eine Vorrichtung zur Befestigung eines alloplastischen Bandes am Knocken, insbesondere am Femur.

Bekannt sind solche Befestigungsvorrichtungen (DE-PS 36 30 390). Befestigungserweiterungen an den Bandenden werden jeweils in in dem Knochen vorzusehende Vertiefungen eingesetzt. Hierbei muß das alloplastische Band um 180" umgelenkt werden. Das Band wird mit Dübel oder Schraube eingeklemmt, wobei die Schraube tief in den Knochen greift . Der Dübel besteht aus Kunststoff . Im übrigen akzeptieren die Patienten in diesem Zusammenhang im allgemeinen heute offene Wunden nicht mehr, sie sind auf arthroskopische Eingriffe fixiert. D.h., die Patienten erwarten, daß die Befestigungen arthroskopisch, d.h. von Innen her, durchgeführt werden können und daß außenseitige zusätzliche Schnittführungen nicht notwendig sind.

Auch die künstlichen Kreuzbänder sollen im Prinzip an der Rinne zwischen den Gelenkknorpeln des Gelenkes sich überkreuzende (je 2) starke, bei jeder Stellung des Knies sich anspannende und damit diese sichernde Bänder, sogenannte Kreuzbänder sein, und nach vorn und hinten zu dem Mittelgrad des Schienbeins sich erstrecken.

Aus der WO 97 29706 ist folgende Bandbefestigung an einem Knochen bekannt: Ein hohler Anker ist mit seinem Außengewinde in den Knochen eingeschraubt. In dem Hohlraum des Ankers ist ein Innenteil gehalten, das außerhalb des Hohlraums einen Kopf als Haltemittel für das Band aufweist.

Schließlich geht aus der WO 97 36557 eine Bandbefestigung an einem Knochen hervor, bei der das Band formschlüssig in einer Hülse gehalten ist, die mit ihrem Außengewinde in das Innengewinde einer Hohlschraube eingreift, wobei das Außengewinde der Hohlschraube in den Knochen eingeschraubt ist.

Der Erfindung liegt die Aufgabe zugrunde, alloplastische Bänder am Knochen, insbesondere am Femur, wirkungsvoller als bisher und insbesondere arthroskopisch befestigen zu können. Erreicht wird dies bei einer Vorrichtung zur Befestigung eines alloplastischen Bandes am Knochen, insbesondere am Femur durch eine Hohlschraube, insbesondere aus Titan mit selbstschneidendem Außengewinde, in deren Innengewinde ein Gewinde einer Hülse einschraubbar ist, wobei das zu befestigende Band an der Innenhülse befestigt ist. Die Hülse kann auch eine Innenschraube sein.

Vorzugsweise ist das Band femoral am Bandende in der Hülse, die in die Hohlschraube einschraubbar ist, verklemmt, wobei zweckmäßig dem Band flächige Klemmmechanismen zugeordnet sind.

Das alloplastische Band ist an einem Ende durch definierte Verklemmung in der Hülse befestigt, wobei am Ende der Hülse ein dem Einschrauben in die Hohlschraube dienender Sechskant festsitzt.

Zur Verklemmung wird eine kleine Hülse auf das Band geklemmt, gegebenenfalls unter Zuhilfenahme definierter Eindrückungen der Hülse gegen das Band. Ist die Hülse auf das Band gepreßt, so wird die Innenhülse darübergeschoben. Bei Zugbelastung erfolgt eine Verklemmung zwischen der kleinen (Klemm)hülse und der Innenhülse. Die Innenhülse wird dann wieder in die Außenhülse geschraubt.

Zur Sicherung gegen eine eventuelle spontane Lockerung zwischen Innenhülse und Titanhohlschraube kann ein Querbolzen in die Innenhülse, insbesondere gegen ihr distales Ende hin, eingebracht sein.

Alternativ kann im Bereich des Sechskants eine Sicherung gegen sponate Lockerung, vorzugsweise als Sicherungsring, angebracht werden.

Am anderen Ende ist zur Befestigung am Knochen eine aufgrund von Reibung funktionierende Einrichtung, insbesondere in Form von Klemmplatten, vorgesehen. Diese Klemmplatten können aufgerauht sein und werden verbunden mit Befestigungsschrauben, die selbstschneidende Außengewinde tragen. Auch können an der Tibia Befestigungen mit herkömmlichen oder modifizierten Staples vorgesehen sein. Tibial kann der Befestigungsvorrichtung eine Zugfeder zugeordnet sein; die Klemmplatten können an beiden Enden konisch aufgeweitet sein, um ein Durchscheuern des Bandes und ein Abknicken zu verhindern. Gegebenenfalls wird das Band auch über Staples geklammert. Geschaffen wird also für das im allgemeinen aus Polyethylenterephthalat bestehende alloplastische an sich bekannte Band eine integrierte Befestigungsvorrichtung zur femoralen Befestigung, wodurch das Band insbesondere im Bereich der femoralen Befestigung, welche als kritischer gilt, eine erhöhte mechanische Festigkeit gegenüber herkömmlichen Systemen aufweist. Die femorale und die tibiale Befestigung erfolgen analog des natürlichen Kreuzbandverlaufes. Die Hülse, an deren einem Ende durch die definierbare Klemmspannung das alloplastische Band befestigt ist, besteht aus Edelmetall, beispielsweise Titan und weist ein Außengewinde zur Verankerung in der Titanhohlschraube auf.

Wichtig ist die Verbindung des Sechskantes mit der Hülse. Der Sechskant dient zum einfachen Einschrauben der Hülse und des alloplastischen Bandes in die Titanhohlschraube mittels eines Hohlsechskantschlüssels, der auf dem Hülsensechskant aufgesetzt wird. Dabei wird in dem Hohlsechskantschlüssel auch das Band geführt.

Das Außengewinde dieser Hülse stellt somit das passende Gegenstück zum Innengewinde der Titanhohlschraube dar und ergänzt somit das gesamte System der femoralen Befestigung zu einer funktionellen Einheit.

Zur Sicherung gegen eine eventuelle spontane Lockerung zwischen Innenhülse und Titanhohlschraube dient ein Querbolzen, der z.B. aus UHMWPE bestehen kann, der in die Innenhülse eingebracht wird. Das Innengewinde der Hohlschraube schneidet beim Einschrauben in ihn ein. Es ergibt sich eine reib- und formschlüssige Verbindung. Der Querbolzen kann gegen das Ende der Hülse hin gesetzt werden. Alternativ kann im Bereich des Sechskants eine Sicherung gegen sponate Lockerung, vorzugsweise als Sicherungsring, angebracht werden.

Das runde alloplastische Band aus Polyethylenterephthalat hat eine Reißkraft von z.B. 4000 N und eine Dehnung, die mit der des natürlichen Kreuzbandes verglichen werden kann. Die Reißkraft des alloplastischen Bandes liegt deutlich über der des natürlichen Kreuzbandes (ca. 1300 N), was zum Vorteil hat, daß das alloplastische Band eine hohe funktionelle Beanspruchung ermöglicht.

In den femoralen Knochen wird die Titanhohlschraube eingeschraubt, in die wiederum die Hülse mit dem geführten alloplastischen Band eingedreht wird. Zur Befestigung im tibialen Bereich werden entsprechende Klemmplatten bzw. "Staples" und/oder Knochenspan verwendet.

Eine mögliche Befestigung tibial sind konisch verlaufende Klemmplatten aus Titan. Diese Klemmplatten werden mittels zwei bzw. vier Schrauben mit selbstschneidendem Außengewinde miteinander verbunden und im tibialen Bereich verankert.

Auch hier ist die tibiale Verankerung mittels Staples möglich, die im Gegensatz zu den herkömmlichen Staples entsprechend modifiziert sein können. Durch den Hohlschraubenzieher im femoralen Bereich, durch dessen Hohlraum das Band gezogen ist und der über einen Außeninbus verfügt, passend zur Innenschraube, kann das gesamte Band mit Innenschraube in der Außenschraube femoral fixiert sein, ohne daß sich das Kunststoffband durch das Einschrauben verdrillt. Der Hohlschraubenzieher greift in die Hülse, die mit Außengewinde in die Titanhohlschraube, das ist die selbstschneidende Schraube, hineingeschraubt wird, bei der ein Sechskant fest mit der Hülse verbunden ist, auf den der Sechskantinnenschlüssel (Hohlschraubenzieher) aufgesetzt wird.

Nachzutragen wäre noch die Definition:
Kreuzband bzw. Band ist ein geringelastisches Bindegewebe. Als künstliches Kreuzband werden üblicherweise solche mit Flechtung benutzt.

Das Band ist in der Hülse definiert verklemmt und durch den Formschlußbolzen, der durch das Innengewinde der Hohlschraube verformt wird, gegen selbständige Lockerung gesichert. Dieser Formschlußbolzen befindet sich im bandfernen Ende der Hülse. Die konischen Aufweitungen der Klemmplatten an den Bandenden dienen der Vermeidung jeder Kerbwirkung.

Zusammengefaßt ergeben sich durch das neuartige System die folgenden Vorteile:
a) es ergibt sich eine gestreckte Position des Implantates bei der Montage ohne kritische Umlenkstellen des alloplastischen Bandes im Bereich der Befestigungen in Funktion;
b) es ergibt sich eine definierte Klemmung bei der Bandbefestigung, insbesondere im kritischeren femoralen Bereich;
c) es ergibt sich eine definierte Vorspannung durch die Befestigungstechnik im tibialen Bereich (evtl. mittels einer Zugfeder);
d) es ergibt sich eine einfache, standardisierte und reproduzierbare OP-Technik in einem begrenzten Zeitrahmen;
e) es ergibt sich eine minimal invasive OP-Technik;
f) es ergibt sich eine sofortige Stabilität des betroffenen Gelenkes und somit frühzeitige Mobilisation, d.h. ohne entsprechende "Ruheschäden";
g) es ergibt sich eine problemlose Wechseltechnik, insbesondere dadurch bedingt, daß die Titanhohlschraube im Knochen verbleibt und zur erneuten Befestigung eines identischen Revisionsbandes dient;
h) scharfe Knochenkanten sind für das alloplastische Band unproblematisch, da die Titanhohlschraube und die Hülse einen Kontakt des Bandes zum Knochen im Bereich der femoralen Befestigung verhindern und im tibialen Bereich die Klemmplatten konisch verlaufen.

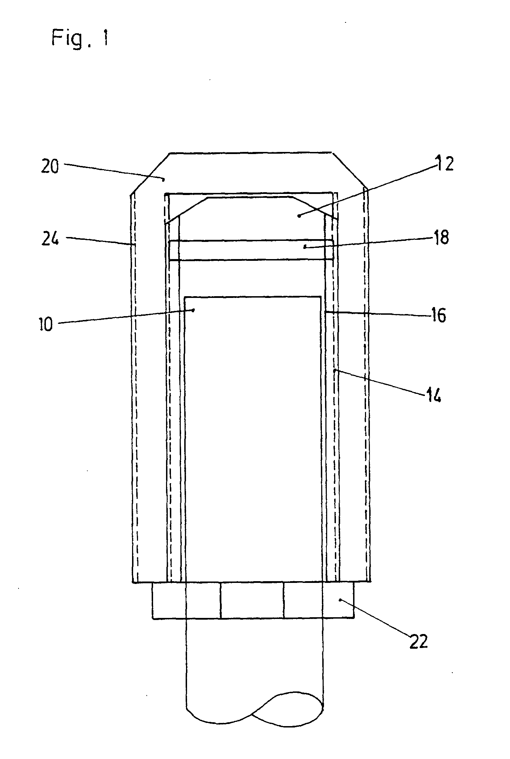

Beispielsweise Ausführungsformen der Erfindung sollen nun mit Bezug auf die beiliegenden Figuren näher erläutert werden, in denen
- Figur 1: eine Befestigungsvorrichtung für ein Kreuzband femurseitig zeigt und
- Figur 2 und 3: Beispiele für Befestigungen tibiaseitig sind.

Das an sich bekannte Band 10 aus Polyethylenterephthalat, gewebt und verstreckt, wird gehalten in einer Innenhülse 12 mit Außengewinde 14. Ein Bolzen 18 ist quer durch das Band 10 etwa zum Ende der Hülse 12, d.h. im bandfernen Bereich, geführt. Der Bolzen 18 besteht aus Kunststoff. Die Innenhülse 12 wird in einer Hohlschraube 20, vorzugsweise aus Titan, eingesetzt bzw. wird in diese eingeschraubt. Beim Einschrauben verformen sich die Enden des vorstehenden Kunststoffbolzens 18, so daß das alloplastische Band einmal durch Klemmung, einmal durch Formschluß gehalten wird. Die Titanhohlschraube 20 besitzt ein Außengewinde 24. Dieses Außengewinde ist selbstschneidend und wird femoral befestigt. Die Titanhohlschraube wird zunächst in den femoralen Knochen eingeschraubt, wobei in die Titanhohlschraube die Hülse mit dem alloplastischen Band eingeschraubt wird oder eingeschraubt ist. Der zylindrische Querbolzen kann beispielsweise aus UHMWPE bestehen. An der Innenhülse 12 ist starr ein Sechskant 22 befestigt. Femorale und tibiale Befestigungen erfolgen analog des natürlichen Kreuzbandverlaufes. Der erwähnte Sechskant 22 dient zum einfachen Einschrauben der Hülse 12 und des alloplastischen Bandes 10 in die Titanhohlschraube 20 mittels eines hier nicht dargestellten Hohlsechskantschlüssels, der einfach auf den Hülsensechskant aufgesetzt wird. Dabei wird in dem Hohlsechskantschlüssel auch das Band geführt.

Außengewindehülse 14 und Innengewindehohlschraube 16 aus Titan sind aufeinander abgestimmt (passen zueinander). Das gesamte System der femoralen Befestigung wird so zu einer funktionellen Einheit verschmolzen. Hülse 12 und Sechskant 22 bilden ein Stück. Neu ist u.a. auch, daß trotz Einschrauben der Hülse das Band 10 nicht mitverdreht wird.

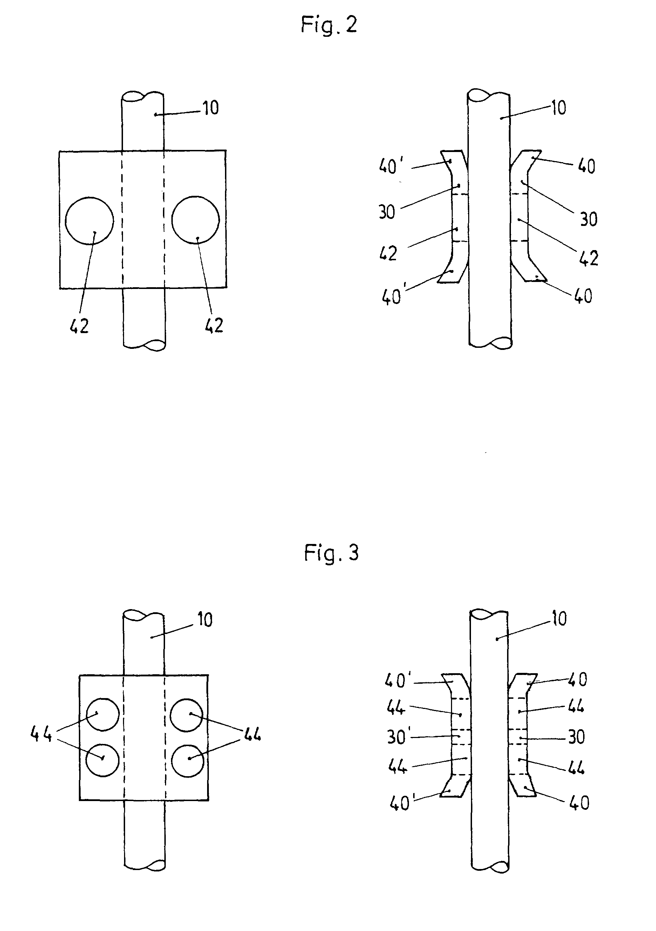

Eine mögliche tibiale Befestigung ist in den Figuren 2 und 3 dargestellt. Das Band 10 wird zwischen zwei Klemmplatten 30, 30', die innen aufgerauht sind, und zwar als Sicherung gegen Durchrutschen, gelegt. Das Material der Klemmplatten 30, 30' ist z.B. Titan. Die beiden Platten werden durch zwei Schrauben 42 (Figur 2) oder vier Schrauben 44 (Figur 3) mit selbstschneidendem Außengewinde miteinander verbunden und im tibialen Bereich verankert. Tibial ist also die Doppelfunktion gegeben: einmal wird das Band 10 über die Schraubenfixation festgelegt, zum anderen wird die Befestigung gegen die Tibia vorgenommen.

Die Figuren 2 und 3 zeigen eine Einzelheit und lassen erkennen, wie die Klemmplatten 30, 30' an den Enden 40, 40' aufgeweitet sind. Dies dient einer Sicherung gegen eine Kerbwirkung der Klemmplatten gegenüber dem Band 10. Die Klemmung erfolgt in jedem Falle im Bereich der Schrauben.

Die linken Seiten der Figuren 2 und 3 zeigen die Draufsicht auf die tibiale Befestigung mit je einer Bohrung 42; 42 bei der Figur 2 und zwei Bohrungen 44, 44; 44, 44 bei der Figur 3. Man erkennt die Oberplatten 30 und die Unterplatten 30'. Rechts ist jeweils die Seitenansicht angegeben. Die Enden 40 der Oberplatten sind hochgebogen, um jede Kerbwirkung zu vermeiden. Die Bohrungen 40 bzw. 42 dienen zur Fixierung am Knochen mittels Schrauben. Diese können selbstschneidendes Außengewinde haben. Dargestellt sind lediglich die Schraubenlöcher 42 bzw. 44.

Als Alternative kann die Unterplatte 30'; 40' fortgelassen werden, d.h. die Verklemmung des Bandes kann direkt am Knochen vorgenommen werden.-Man kann dies auch als "modifiziertes Staple" bezeichnen.

Figur 3 zeigt das Gleiche mit zwei Bohrungen, durch welche die (nicht gezeigten) Schrauben von der Oberplatte zur Unterplatte gehen und das Band 10 hierzwischen klemmen.

In Figur 3 dienen zwei Bohrungen zur Fixierung am Knochen mittels Schrauben. Diese können wieder selbstschneidendes Außengewinde haben.

Nach einer alternativen, hier nicht dargestellten Ausführungsform, kann die Unterplatte fortgelassen werden, d.h. die Verklemmung erfolgt direkt am Knochen. Auch hier handelt es sich um ein sog. modifiziertes Staple.

Eine alternative Befestigung, welche aufgrund der Bekanntheit nicht dargestellt ist, stellen sog. "Staples" dar. Dabei handelt es sich um eine Befestigungsklammer bzw. "Staple" mit vier Beinen, die in die Tibia eingetrieben wird. Diese Klammer besteht wieder aus Edelmetall oder Titan. Ein Umbiegen der Enden im Knochen ist nicht erforderlich. Die Staples mit ihren Schenkeln oder Beinen können gegenüber den bekannten Staples entsprechend modifiziert sein.

Zur femoralen Befestigung wird also ein Hohlschraubenzieher mit einem Außeninbus verwendet, der zur Innenschraube paßt. Interessant ist, daß das gesamte Band mit Innenschraube in der Außenschraube femoral fixiert werden kann, ohne daß sich das Kunststoffband durch Eindrehen verdrillt und ohne daß Spannungen auf den Knochen ausgeübt werden. Falls, was an sich nicht vorgesehen ist, das Band ersetzt werden soll, ist hier ebenfalls eine einfache Möglichkeit gegeben.

## Patentansprüche

1. Vorrichtung zur Befestigung eines alloplastischen Bandes (10) am Knochen, insbesondere am Femur, mit einer Hohlschraube (20) mit selbstschneidendem Außengewinde (24) und mit einem Innengewinde, in das eine mit einem Außengewinde (14) versehene Innenhülse (12) einschraubbar ist, wobei das zu befestigende Band (10) an der Innenhülse (12) befestigt ist, **dadurch gekennzeichnet, dass** auf das in der Innenhülse (12) aufgenommene Bandende (10) eine Klemmhülse geklemmt ist und dass das Band (10) an der Innenhülse (12) so befestigt ist, dass bei Zugbelastung das Band (10) zwischen der Klemmhülse und der Innenhülse (12) verklemmt, wobei fest am Ende der Innenhülse (12) ein dem Einschrauben in die Hohlschraube (20) dienender Sechskant (22) sitzt, der auf seiner Innenseite zur Vermeidung kritischer Umlenkungen des Bandes (10) verrundet ist.

2. Vorrichtung nach Anspruch 1, **dadurch gekennzeichnet, dass** der Werkstoff der Hohlschraube (20) Titan ist.

3. Vorrichtung nach Anspruch 1 oder 2, **dadurch gekennzeichnet, dass** dem Band (10) flächige Klemmmechanismen zugeordnet sind.

## Claims

1. Device for fastening an alloplastic ligament (10) to the bones, particularly to the femur, comprising a hollow screw (20), having a self-tapping external thread (24) and having an internal thread, into which an inner sleeve (12) having an outer thread (14) can be screwed, the ligament (10) being fastened to the inner sleeve (12), **characterised in that** upon the ligament end received in the inner sleeve (12) a clamping sleeve is clamped and that the ligament (10) is fastened to the inner sleeve (12) such that on applying tension to the ligament (10) is clamped between the clamping sleeve and the inner sleeve (12), firmly at the end of the inner sleeve (12) a hexagon (22) is arranged serving screwing into the hollow screw (20) which hexagon on its inner side for avoiding critical deflections of the ligament (10) is rounded.

2. Device according to claim 1, **characterised in that** the material of the hollow screw (20) is titanium.

3. Device according to claims 1 or 2 **characterised in that** flat clamping mechanisms are associated to the ligament (10).

## Revendications

1. Dispositif pour fixer un ligament alloplastique (10) à l'os, particulièrement au Fémur avec une vis creuse (20) ayant un filetage extérieur auto taillante (24) et avec un taraudage, dans lequel peut être vissé un manchon intérieur (12) ayant un filetage extérieur (14), le ligament à fixer (10) étant fixé au manchon intérieur (12), **caractérisé en ce qu'**une douille de serrage est serré au bout du ligament (10) relevé dans le manchon intérieur (12) et en ce que le ligament (10) est fixé au manchon intérieur (12) telle que le ligament (10) dans le cas d'une contrainte de tension est serré entre la douille de serrage et le manchon intérieur (12), un hexagone (22), qui se trouve au bout du manchon intérieur (12), étant arrondi à sa face intérieur pour éviter des renvois critiques.

2. Dispositif selon la revendication 1, **caractérisé en ce que** le matériel de la vis creuse (20) est du titane.

3. Dispositif selon la revendication 1 ou 2, **caractérisé en ce que** des mécanismes méplats de serrage sont associées au ligament (10).
